(19) Europäisches Patentamt
European Patent Office
Office européen des brevets

(11) Publication number: 0 055 857
A2

# (12) EUROPEAN PATENT APPLICATION

(21) Application number: 81110825.7

(22) Date of filing: 29.12.81

(51) Int. Cl.[3]: A 61 K 7/42
A 61 K 9/70, A 01 N 25/10
C 08 K 5/00

(30) Priority: 30.12.80 US 221504

(43) Date of publication of application:
14.07.82 Bulletin 82/28

(84) Designated Contracting States:
AT BE CH DE FR GB IT LI NL SE

(71) Applicant: UNION CARBIDE CORPORATION
Old Ridgebury Road
Danbury Connecticut 06817(US)

(72) Inventor: Leung, Pak Sang
15 Woodland Road
Highland Mills New York 10930(US)

(72) Inventor: Goddard, Errol Desmond
349 Pleasant Lane
Haworth New Jersey 17641(US)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

## (54) Potentiation of topical compositions.

(57) Topically applied compositions are disclosed. Further, sunscreening compositions are disclosed comprising ultraviolet light-absorbing materials and film-forming polymers which exhibit enhanced protection from erythema-causing radiation. The observed enhancement in activity of the topical composition may be seen to occur with other topically applied materials.

EP 0 055 857 A2

PATENTANWÄLTE
WUESTHOFF-v.PECHMANN-BEHRENS-GOETZ
EUROPEAN PATENT ATTORNEYS

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING.; DIPL.-WIRTSCH.-ING. RUPERT GOETZ

0055857

EP-55 423
12,914



D-8000 MÜNCHEN 90
SCHWEIGERSTRASSE 2

## FIELD OF THE INVENTION

The instant invention relates to improved topically applied compositions comprising topical material and film-forming polymer. Preferred topical materials are ultraviolet light-absorbing materials.

## BACKGROUND OF THE INVENTION

The use of topically applied compositions finds application in numerous areas ranging from cosmetic preparations and anesthetics to insecticides and dyes. The varied use of topically applied materials has led to intense interest in minimizing the amount of material employed for a particular application while yet achieving the same desired effect. As a result of this intense interest, the search for ways to improve the effect of a fixed amount of topical material has been of prime importance in all areas wherein topical materials are employed. An example of this interest may be found in the prior art relating to sunscreening compositions.

The use of sunscreening compositions is required by a large segment of society since only a small portion of those exposed to sunlight have the natural pigmentation which provides protection against the harmful effects of solar radiation. Because of this propensity of many people to easily show erythema under prolonged exposure to sunlight, there is a need for sunscreening compositions to protect against erythema-causing radiation, i.e. ultraviolet radiation in the region of 260 to 320 nanometers, so that longer exposure to the sunlight with less risk of sunburn is possible.

A variety of sunscreening compositions are known in the art. The tendency in formulating sunscreening compositions has been to prepare compositions which are water-resistant or "substantive" to the skin, e.g., by chemically modifying the ultraviolet absorber to increase its interaction with the skin by quaternizing imidazoles as described in U.S. Pat. No. 3,506,758; by copolymerizing ultraviolet light absorbing monomers with other monomers to form water-resistant films (see, for example U.S. Patent Nos. 3,529,055 and 3,864,473); or by forming polymeric films with water-insoluble polymers (e.g. see, U.S. Patent 3,784,488).

The use of the acid form of crosslinked ethylene-maleic anhydride copolymers to retain ultraviolet light absorbers is disclosed in U.S. Patent No. 3,821,363. The use of a water insoluble acrylate polymer having a solubility parameter of 6 to 10 in weak hydrogen bonding solvents is disclosed in U.S. Patent No. 4,172,122. The use of water-insoluble, alcohol-soluble, film-forming polyamide materials is disclosed in U.S. Patent No. 3,895,104 solely for the purpose of providing improved substantivity.

The sunscreening compositions of the prior art have not heretofore utilized film-forming polymers to enhance the effectiveness of topically applied material with which they are employed, although varied uses, as above described have occurred. Thus, the purpose of employing polymers or polymeric materials in the compositions of the prior art has been directed solely towards improving the adherency, i.e., substantivity, of the topical material to the skin or have been employed solely as thickening agents.

The use of film-forming polymers has been suggested as being beneficial in potentiating sunscreens. In the brochure entitled "Polymer JR for Skin Care" by Union Carbide Corporation (dated June 1977) it is disclosed that Polymer JR (a trade mark of Union Carbide Corporation and hereinafter described) solution of two sunscreens (homomenthyl salicylate and p-amino benzoic acid (PABA) showed increased protection from erythema-causing radiation. The degree of enhancement in protection shown in this brochure is minimal (about 5% by weight p-amino benzoic acid and about 8% by weight homomenthyl salicylate in admixtures with about 1% by weight and 2% by weight, respectively, Polymer JR (hereinafter defined)).

Further, the use of polymers in hair care compositions has been widely suggested and, further, has been employed to increase particle deposition and retention of particulate substances on a surface (U.S. Patent No. 3,580,853).

Although the aforementioned prior art disclosed the utility of polymers for a number of various purposes the prior art does not disclose the use of a film-forming polymer in conjunction with a topically applied material wherein the ratio of film-forming polymer to topical material is from about 0.5:1 to about 20:1, preferably from about 1:1 to about 10:1 and most preferably from about 1:1 to 4:1, and wherein the topical material exhibits enhanced behavior as compared to the behavior of the topical material when employed in the absence of the film-forming polymer.

The compositions of this invention employ "film-forming polymers" to "potentiate" topically

applied materials, e.g., ultraviolet light-absorbing materials, contained therewith. The improved effectiveness achieved by employing such film-forming polymers has not heretofore been disclosed or appreciated in the prior art.

The potentiation of topically applied materials provides for more effective and economical use of such materials. In particular, the invention provides improved sunscreening compositions having an effective amount of at least one ultraviolet light-absorbing material and an effective amount of a film-forming polymer wherein when employed in combination with the film-forming polymer the ultraviolet light-absorbing material exhibits improved sunscreening protection as compared to that observed when employed without such film-forming polymer.

DESCRIPTION OF THE INVENTION

It has been found that the addition of one or more film-forming polymer to a composition containing a topical material which is to be topically applied significantly improves the effectiveness of a given amount of said topically material when the ratio of film-forming polymer to topical material is from about 0.5:1 to about 20:1, preferably from about 1:1 to about 10:1, and most preferably from about 1:1 to about 4:1 In particular, in sunscreening compositions, surprisingly, it has been found that the combination of a film-forming polymer and ultraviolet light-absorbing material provides a sunscreening composition that when applied to the skin is dry to the touch without loss of the effectiveness of the ultraviolet light absorbance of the ultraviolet light-absorbing material.

Further, it has been found that the addition of a film-forming polymer to an ultraviolet light-absorbing material has additional beneficial effects on the ultraviolet light-absorbing material in that the peak absorbance of the material may be shifted to more favored wavelengths. Thus, the resulting sunscreening composition as applied to the skin may exhibit ultraviolet light absorbance superior to that exhibited by employing the ultraviolet light-absorbing material without a film-forming polymer.

The compositions of this invention contain topical materials and film-forming polymers and provide enhanced effectiveness for a given amount of topical material employed. For example, the sunscreening compositions of this invention require substantially less ultraviolet light-absorbing material, as compared to commercial sunscreening compositions, so that generally less than about 3 percent by weight of the sunscreen composition will be ultraviolet light-absorbing material preferably from about 0.1 to about 3.0%.

The term "potentiation" is used herein to describe the ability of a film-forming polymer to enhance the effectiveness of topical materials, e.g., ultraviolet light-absorbing materials. The measure of enhancement is based in general on the improved or increased effectiveness of the topical material based on its anticipated use. The measure of enhancement for an ultraviolet light-absorbing material is determined by the ability of a given amount of dried ultraviolet light-absorbing material to absorb a greater amount of ultraviolet light in the presence of the film-forming polymer than when said film-forming polymer is not present, i.e., increased ultraviolet absorbance for a given amount of topical material.

The term "topical materials" as used herein refers to materials which are applied to a given surface to provide a beneficial effect as a result of the activity of the material on said surface. Typical surfaces include, but are not limited to, human skin, plants, plastics, glasses and the like.

The term "topical material" includes, but is not limited to, insecticides, pesticides, ultraviolet light-absorbing materials, anesthetics, fungicides, antimicrobial agents, and the like.

The term "ultraviolet light-absorbing material" (hereinafter "UV-absorbing material") as used herein refers in general to any compound or combination of compounds capable of absorbing ultraviolet light from about 200 to about 370 nanometers. The preferred UV-absorbing materials are those which are capable of absorbing ultraviolet light in the erythemal range from about 260 to about 320 nanometers which may be employed in sunscreening compositions intended for application to human skin. The preferred ultraviolet absorbance of these UV-absorbing materials is in the range 290 to 310 nanometers. UV-absorbing materials suitable for use in this invention include, but are not limited to, para-amino benzoic acid, ethyl ester of para-amino benzoic acid and other esters of para-amino benzoic acid, e.g., the glyceryl ester, esters of substituted para-amino benzoic acid, e.g., amyl isoamyl, or ethyl hexyl esters of para dimethyl aminobenzoic acid, the ethyl esters of para diethyl aminobenzoic acid and esters of paramethoxycinnamic acid, e.g., 2-ethoxyethyl ester, certain esters of salicyclic acid, e.g., homo menthyl salicylate, certain benzophenone derivatives, e.g., 2-hydroxy-4-methoxy benzophenone or 2,2-dihydroxy-4-methoxy benzophenone, mixtures thereof and the like.

The preferred UV-absorbing materials are those which are crystalline materials at below about 200°C. although liquid UV-absorbing materials may be employed. A particularly preferred class of UV-absorbing materials is para-amino benzoic acid (hereinafter PABA) and esters thereof.

In addition to the aforementioned UV-absorbing materials, this invention may permit, surprisingly, the use of materials having their absorption at the lower end of the range of 260 to 320 nanometers. Thus, when materials not generally as desirable as sunscreeners are employed in combination with a film-forming polymer they may exhibit a shift in the range at which they absorb ultraviolet radiation so as to provide sunscreening compositions with an improved wavelength at which absorbance occurs. The shift in absorption from the lower end of the wavelength range, often into the preferred range of about 290 to about 310 nanometers, may permit the use of materials which are not traditionally used as UV-absorbing materials. These materials may also be substantially cheaper to use than conventional UV-absorbing materials.

The term "film-forming polymer" as used herein refers to film-forming polymers that possess the ability

to provide some physical bonding, preferably polar bonding, with the topical material through physical forces, e.g. dipole-dipole, hydrogen bonding, dipole-induced dipole and the like.

In addition, these film-forming polymers tend to form substantive films when applied to the various surfaces and this is particularly the case when applied to human skin.

Although the exact mechanism by which potentation occurs is not known, it is believed to involve the formation of a uniform microdispersion of the topical material in the film-forming polymer as a polymeric film is cast on a given surface. In the case of a crystalline topical material the formation of a uniform microdispersion is evidenced by the virtual absence of the melting point of the topical material when measured in the compositions of this invention (as measured by Differential Scanning Calorimetry (DSC)).

The choice of film-forming polymer is not narrowly critical and may comprise water soluble or water insoluble film-forming polymers. It is preferred to employ water soluble polymers including cellulose derivatives, particularly quaternary nitrogen-containing cellulose ethers, hydroxyethyl cellulose, hydroxypropyl cellulose and hydroxyethyl alkali metal carboxylalkyl cellulose derivatives, and free acid hydroxyalkyl carboxyalkyl cellulose derivatives, as well as vinylpyrrolidone homopolymers and copolymers, polycarboxylic acid derivatives, polyacryamides, vinyl methyl ether homopolymers and copolymers, ethylene oxide resins, and the like.

Water insoluble polymers suitable for this invention include, but are not limited to, water insoluble polyamide polymers, esters of polymeric carboxylic acids, e.g., polyacrylate polymers, polypropylene oxide and derivatives thereof and the like.

The preferred quaternary nitrogen-containing cellulose ethers (hereinafter referred to as QNCC ethers) employed in the topical compositions of this invention are those described in U.S. Patent No. 3,472,840, granted to Stone et al. on October 14, 1969, which disclosure is incorporated by reference herein.

The preferred cellulose ether derivative from which the quaternary nitrogen containing cellulose ethers described above are prepared include those which are water soluble, non-ionic, lower alkyl or hydroxy alkyl substituted. Such derivatives include methyl cellulose, ethyl cellulose, and hydroxyethyl cellulose.

A particularly efficacious quaternary nitrogen-substituted cellulose derivative for the purpose of this invention is available from Union Carbide under the code designation "Polymer JR." This polymer has a molecular weight within the range of from about 100,000 to about 3,000,000. Polymer JR is a cationic cellulose ether have the structure:

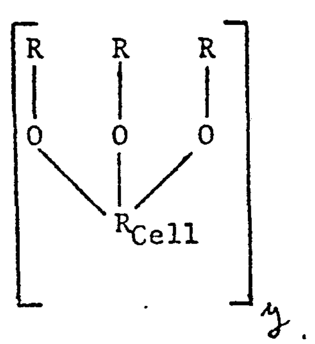

wherein $R_{Cell}$ is a residue of an anhydroglucose unit, wherein y is an integer having values from about 50 to about 20,000 and wherein each R individually represents a substituent of the general formula:

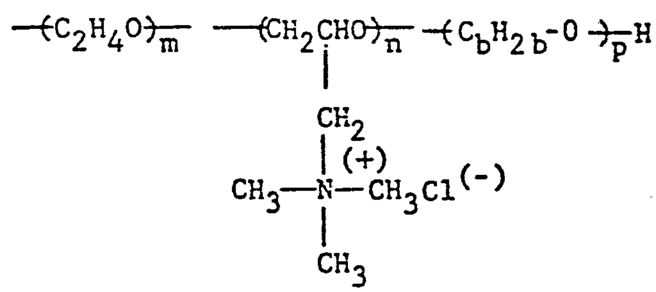

wherein the m is an integer having values from 0 to 10, n is an integer having values from 0 to 3, p is an integer having a value from 0 to 10 and b is an integer having a value from 0 to 3. The average values per anhydroglucose unit are: n is from about 0.1 to about 0.5 and the sum m + p is from about .8 to about 2.

The preferred QNCC ethers for use in the practice of the instant invention are those having viscosities of 50 to 35,000 mPa·s centiposes (cps.) at 25°C. in 2 percent by weight aqueous solutions, when measured by ASTM D-2364-65 (Model LVF Brookfield, 30 rpm Spindle No. 2). QNCC ethers which are particularly useful in the practice of this invention are those sold by Union Carbide Corporation under the trade designation JR-125, JR-400, and JR-30M, signifying a polymer of the type described having viscosities (2 percent solutions by weight) of about 125 mPa·s cps., about 400 mPa·s cps. and about 30,000 mPa·s cps., respectively.

The term "ethylene oxide resins" as used in the instant invention encompass not only the homopolymer, poly(ethylene oxide), but also copolymers of ethylene oxide

in which ethylene oxide is copolymerized with other alkylene oxides such as propylene oxide, butylene oxide, styrene oxide and the like and other comonomers copolymerizable with ethylene oxide including ethylene oxide having terminal groups as ethers or esters, e.g. stearates, and the like. These resins may be water soluble or water insoluble.

Examples of hydroxyalkyl carboxyalkyl celluloses include hydroxyethyl carboxymethyl cellulose, hydroxyethyl carboxyethyl cellulose, hydroxymethyl carboxymethyl cellulose, hydroxypropyl carboxymethyl cellulose, hydroxypropyl carboxyethyl cellulose, hydroxypropyl carboxypropyl cellulose, hydroxybutyl carboxymethyl cellulose, and the like. The preferred alkali metal salts of these hydroxyalkyl carboxyalkyl celluloses are the sodium and the potassium derivatives.

The concentration of topical material may vary but is generally from about 0.01 to about 10.0 percent by weight, preferably is from about 0.1 to about 5.0 percent by weight and most preferably from about 0.1 to about 3.0 percent by weight. The concentration of film-forming polymer is generally employed in an amount from about 0.01 to about 10.0 percent by weight, preferably is from about 0.1 to about 5.0 percent by weight and most preferably from about 0.1 to about 3.0 percent by weight.

The ratio of film-forming polymer to topical material is from about 0.5:1 to about 20:1, preferably from about 2:1 to about 4:1. It has been found that the use of such relative proportions of the topical material and film-forming polymer is important in obtaining a uniform microdispersion and the optimum potentiation of this invention.

When the topical material is an UV-absorbing material, sunscreening compositions of this invention generally contains from about 0.1 to about 5.0 percent by weight ultraviolet light-absorbing material, the preferred range being from about 0.1 to about 3.0 percent by weight and the most preferred range being from about 0.5 to about 2.0 percent by weight. The film-forming polymer is generally present in an amount from about 0.1 to about 10.0 percent by weight, preferably in the range from about 0.1 to about 3.0 percent by weight. The ratio of film-forming polymer to UV-absorbing material is generally from about 0.5:1 to about 20:1, preferably from about 1:1 to about 10:1, and most preferably from about 1:1 to 4:1.

In addition, the compositions of the invention may additionally comprise a wide range of inert and active ingredients commonly employed in cosmetic, toiletries, and other preparations, and include perfumes, colorants, humectants, emollients, skin conditioners, solvents, propellants, pigments, fillers, diluents, depilatories, stabilizers, and the like.

Sunscreening compositions were prepared in accordance with this invention and were tested both in-vivo and in-vitro in ultraviolet (UV) absorption

In carrying out the in-vivo study, a Rofin arc lamp 7823 system with a 150 W Xenon lamp was used as the UV source. The UV rays were focused by a glass lens of focal length 21 mm and were filtered by a two inch path length of water in a quartz cell to remove most infra-red radiation and to

cut down the heat. A secondary filter (Schopp UG-5) was used to eliminate light with a wavelength longer than about 400 nm and shorter than about 240 nm. The energy of the UV output was calibrated with a thermopile supplied by the Eppley Laboratory. It was found that at 40 cm from the focus lens a 30second irradiation time was the exposure needed to deliver three times the minimal erythemal dosage (MED). It was later found that irradiation from a 1 $cm^2$ circular opening at 30 cm from the focus lens for 30 seconds was most discriminating. In all the in-vivo studies, the sunscreen preparation was applied at a level of 5 microliter on 1 $cm^2$ skin.

In-vitro studies were carried out by measuring the UV absorbance of various sunscreening compositions of this invention after said same had been dried on planar quartz plates with a Beckman Acta V spectrometer.

Topical compositions were prepared according to this invention containing topical materials comprising other than UV-absorbing materials. These compositions were examined by Differential Scanning Calorimetry (DSC) using a DuPont 900 Thermal Analyzer. A loss in crystallinity of the topical material observed in these compositions is consistent with the presence of these materials in a uniform microdispersion in the dry cast films.

In carrying out the several examples, hereinafter, the following film-forming polymers were employed:

CELQUAT L200 (CELQUAT is the Trade Mark of National Starch Corp.) is a quaternary ammonium cellulose

derivative.

MERQUAT 100 (MERQUAT is the Trade Mark of Merck and Company, Inc. Rahway, New Jersey) and CALGON 502 (CALGON is the Trade Mark of Calgon Corp.) are both polymers of N, N-dimethyl 3,5-methylene piperidinium chloride.

GAFQUAT-755 (GAFQUAT is the Registered Trade Mark of GAF Corporation) is a quaternary ammonium compound which is a copolymer of vinylpyrrolidone and dimethyl amino ethyl methacrylate quaternized with diethyl sulphate. The various GAFQUATS have molecular weights in the range of from about 100,000 to about 1,000,000 with GAFQUAT-755 having a molecular weight of about 1,000,000 (available as a 20% by weight aqueous solution);

POLYOX (POLYOX is the Trade Mark of Union Carbide Corporation) designates a group of very high molecular weight polymers of ethylene oxide (PEO).

CARBOWAX (CARBOWAX is the Trade Mark of Union Carbide Corporation) for polyethylene glycol polymers. CARBOWAX 6000 and CARBOWAX 20M are code names for polymers having molecular weights of about 6000 and 20,000, respectively;

METHOCEL (METHOCEL is the Trade Mark of Dow Chemical Corporation) are methyl ethers of cellulose, including hydroxypropyl methyl cellulose;

CELLOSIZE (CELLOSIZE is the Trade Mark of Union Carbide Corporation) is a hydroxyethyl cellulose.

CELLOSIZE (QP-300H) is a grade of hydroxyethyl cellulose polymer.

KESSCO PEO 6000 (KESSCO is the Trademark of Armour) is the Trade Mark for a group of polymers which are polyethylene glycol esters of fatty acids;

KLUCEL L (KLUCEL is the Trade Mark of Hercules Corporation) is a hydropropyl cellulose polymer;

Polyvinyl alcohol (PVA) (100 percent hydrolyzed) having a molecular weight of about 14,000, sold by Aldrich Chemical Company;

Propylene glycol (PPG 1025) having a molecular weight of about 1025, sold by Union Carbide Corporation;

GANTREZ (GANTREZ is the Trade Mark of GAF Corporation) is an ester of polymethyl vinyl ether of maleic acid; and

CROTEIN Q (sold by Croda Corporation) is a cationic protein.

The compositions of the invention may be further illustrated by the following nonlimiting examples. Ingredients used in the compositions are identified by commercial designation as hereinbefore set forth.

EXAMPLE 1

To test the sunscreening compositions of this invention and the potentiating effect of water-soluble film-forming polymers on UV-absorbing materials the following experiment was carried out.

Four test cases were evaluated using para-amino benzoic acid and Polymer JR, as follows:

(1) Human skin was irradiated with Rofin Xenon lamp filtered radiation after the application of 5 microliter/

$cm^2$ of 1% by weight of para-amino benzoic acid (PABA) in 50 percent ethanol;

(2) Human skin was irradiated with radiation, as in (1), after the application of 5 microliter/$cm^2$ of a solution containing 1 percent by weight Polymer JR and 1 percent by weight PABA in 50 percent ethanol;

(3) Human skin was irradiated, as in (1), with radiation through a Polymer JR filter (prepared by drying a 1 percent by weight Polymer JR solution on a quartz plate) after the application of 5 microliter/$cm^2$ of 1 percent by weight PABA to the skin; and

(4) Human skin was irradiated with radiation, as in (1), through a filter prepared by applying 5 microliter/$cm^2$ of a solution containing 1 percent by weight Polymer JR and 1 percent by weight PABA in 50 percent ethanol on a quartz plate and drying it thereon.

Evaluation of the sunscreening solutions of Cases (1) and (3) (not of this invention) revealed that erythema was observed whereas, no sign of erythema was observed for Cases (2) and (4). Thus, when the sunscreening compositions of this invention were employed Cases (2) and (4)) the UV-absorbing material clearly showed improved protection from erythema, i.e. potentiation of the topical material p-amino benzoic acid.

EXAMPLES 2-13

Sunscreening compositions prepared according to the invention were evaluated by in-vitro UV absorption studies by drying on a 3 cm square quartz plate 20 microliter solutions containing 0.5 weight percent PABA and a film-forming polymer. The UV-absorbing material selected herein was PABA. (The concentration of polymers as indicated by Polymer:PABA ratio in Table I).

Examples 2-13 of Table I show the potentiation achieved by employing various film-forming polymers with PABA when employed in a ratio of film-forming polymer to UV-absorbing material of from about 0.5:1 to about 4:1. The UV absorbance of para-amino benzoic acid when measured in the absence of a film-forming polymer, applied in 50 percent ethanol, after drying is 0.06.

The aforementioned compositions were examined by microscopy. The crystal formation of dried para-amino benzoic acid was observed to change when a film-forming polymer was employed. As compared to PABA, which is dried in the absence of a film-forming polymer, the addition of such a polymer led to a significant reduction in the degree of crystallinity of the dried PABA. Differential Scanning Calorimetry showed the melting point of PABA was substantially eliminated, consistent with the formation of a uniform microdispersion of UV-absorbing material.

The results of Table I are graphically display- the Figure. The absorbance as a function of the ratio (by weight) of film-forming polymer to p-amino benzoic acid can be seen by reference to the Figure wherein the letters A through L represent compositions containing the following film-forming polymers in admixture with p-amino benzoic acid:

| Letter in Fig. | Film-Forming Polymer |
|---|---|
| A | GAF QUAT 755N |
| B | PVA 1400 |
| C | MERQUAT 100 |
| D | CELLOSIZE (QP 300H) |
| E | JR 125 |
| F | KLUCEL L |
| G | PEO 20M |

| Letter in Fig. | Film-Forming Polymer |
|---|---|
| H | CELQUAT L200 |
| I | KESSCO PEO 6000 (STEARATE) |
| J | CARBOWAX 6000 |
| K | JR 400 |
| L | PPG(1025) |

EXAMPLE 14

The effect on the peak wavelength at which an UV-absorbing material absorbs when cast as a dry film in the presence of a film-forming polymer was evaluated as a function of the pH of a composition containing a UV-absorbing material and a film-forming polymer.

A sunscreening composition according to the invention was prepared comprising $0.83 \times 10^{-3}$ percent by weight PABA and $0.83 \times 10^{-3}$ percent by weight Polymer JR-400. The UV absorbance of this composition was tested after adjusting the pH of the three samples of the composition such that the composition had a pH of 10.5, 8.1 and 4.1 (A 1 percent by weight solution of PABA (50 percent ethanol) has a pH of 4.2). The peak absorbance of these solutions was measured and showed absorbance at about 260 nm, about 265 nm and about 285 nm, respectively (measured in a 1 cm cell). To demonstrate this effect of drying of the solutions containing a UV-absorbing material and a film forming polymer a second series of sunscreening composition were prepared comprising 0.5 percent by weight PABA and 0.5 percent by weight Polymer JR 400 in 50 percent ethanol. This series of samples were prepared having pH 10.5, 8.2 and 4.4. Twenty microliters of each applied to a quartz plate ($3cm^2$) and dried. The peak absorbance of each sample was 260 nm, 270 nm and 295 nm respectively. Thus, a shift in the UV-absorbing material was observed upon formation of a dry film.

TABLE I (1,2)

| Example | Film-Forming Polymer (Polymer:PABA Ratio) | Absorbance (3,4) 0.5:1 | 1:1 | 1.5:1 | 2:1 | 3:1 | 4:1 |
|---|---|---|---|---|---|---|---|
| 2 | GAF QUAT 755N | 0.8 | 2.0 | 2.6 | 2.5 | - | - |
| 3 | PVA 1400 | 0.8 | 0.8 | 0.9 | 1.2 | 2.5 | - |
| 4 | MERQUAT 100 | 0.28 | 0.7 | 1.7 | 1.65 | 2.3 | - |
| 5 | CELLOSIZE (QP 300H) | 0.3 | 1.05 | 2.2 | 2.8 | 1.8 | - |
| 6 | JR 125 | 0.8 | 1.7 | 2.0 | 2.7 | 1.9 | - |
| 7 | KLUCEL L | 0.4 | 0.5 | 1.4 | 1.8 | 1.5 | - |
| 8 | PEO 20 M | 0.55 | 1.9 | 2.1 | 2.8 | 1.8 | - |
| 9 | CELQUAT L 200 | 1.0 | 1.1 | 2.5 | 2.5 | 1.5 | - |
| 10 | KESSCO PEO 6000 | 0.3 | 1.0 | 1.5 | 2.3 | 1.5 | - |
| 11 | CARBOWAX 6000 | - | 0.25 | - | 1.4 | - | 1.4 |
| 12 | JR 400 | 0.5 | 0.9 | 2.3 | 2.3 | 1.3 | - |
| 13 | PPG (1025) | 0.22 | 0.35 | 0.82 | 1.5 | 1.1 | - |

(1) Dry films cast from 20 microliters of a solution (50 percent ethanol containing 0.5 percent by weight PABA and polymer (concentration as indicated by ratio) on a planar quartz surface with an area of 3 $cm^2$.

(2) Analysis by DSC showed a substantial loss of crystallinity of PABA and virtual elimination of the melting point of PABA with increasing ratio of film-forming polymer to PABA.

(3) Absorbance = log $\left(\frac{1}{\text{Transmittance}}\right)$; measured at 296 nm.

(4) The absorbance of PABA; measured after drying PABA on a planar quartz surface, prepared by applying 20 microliters of a solution (50% ethanol containing 0.5% by weight PAPA) on a planar quartz surface having an area of 3 $cm^2$ was 0.05

EXAMPLE 15-19

The effect on UV absorbance of PABA of a change in the molecular weight of the film-forming polymer was tested by preparing three sunscreening compositions according to this invention as follows:

(1) 0.5 percent by weight PABA and 1.0 percent of Polymer JR 125;

(2) 0.5 percent by weight PABA and 1.0 percent by weight Polymer JR 400; and

(3) 0.25 percent by weight PABA and 0.5 percent by weight Polymer JR 30M.

The aforementioned sunscreening compositions were evaluated for UV absorbance in the range from about 250 nm to about 320 nm with peak absorbancy being at about 296 nm. The measured ultraviolet light absorbance is shown in Table II.

TABLE II

| Example | PABA Conc.(2) | Polar Polymer | Polymer Conc | Absorbance(1) |
|---|---|---|---|---|
| 15 | 0.5 | JR-125 | 1.0 | 2.5 |
| 16 | 0.5 | JR-400 | 1.0 | 2.3 |
| 17 | 0.25 | JR-30M | 0.5 | 1.2 |
| 18 | 5.0 | - | - | 0.1 |
| 19 | 1.0 | - | - | 0.06 |

(1) UV absorbance at 296 nm.

(2) Weight percent

Table II shows that the UV absorbance of the sunscreening compositions changes little as a function of the molecular weight of the film-forming polymer. Further, Table II shows the marked increase in UV absorbance, as measured in-vitro studies, of the sunscreening compositions of this invention over a 5.0 percent and a 1.0 percent by weight PABA solution (in 50 percent ethanol) when employed without film-forming polymer.

EXAMPLES 20-28

Sunscreening compositions were prepared according to this invention comprising 0.25 percent by weight ESCALOL 507, i.e. 2-ethyl hexyl para-dimethyl amino benzoate (ESCALOL 507 is the Trade Mark of Van Dyke Corporation) and from about 0.25 to about 1.25 percent by weight of a film-forming polymer, as shown in Table III.

TABLE III (1,2)

| Example | Film-Forming Polymer | Ratio of Polymer: ESCALOL | Absorbance |
|---|---|---|---|
| 20(3) | - | - | 0.08 |
| 21 | CELLOSIZE (QP 300H) | 1/1 | .15 |
| 22 | CELLOSIZE (QP 300H) | 2/1 | .15 |
| 23 | CELLOSIZE (QP 300H) | 3/1 | .15 |
| 24 | CELLOSIZE (QP 300H) | 4/1 | .15 |
| 25 | PEO (20M) | 1/1 | 1.35 |
| 26 | PEO (20M) | 1/5 (4) | 1.2 |
| 27 | PEO (20M) | 4/1 | 0.85 |
| 28 | PEO (20M) | .5/1 | 1.3 |

(1) UV Absorbance at 296 nm.

(2) Dry films cast from 20 microliter of a solution (50 percent ethanol) 0.25 percent by weight ESCALOL and polymer (concentration as indicated by ratio) on 3 $cm^2$ quartz surface.

(3) Comparative Example (No film-forming polymer present).

(4) Surprisingly, it was discovered that PEO (20M) and ESCALOL form a synergistic combination when employed in an amount such that the ratio by weight is at least about 1:10 of PEO (20) to ESCALOL and not greater than about 20:1, preferably 1:5 to about 4:1.

EXAMPLES 29-33

The compositions of Table IV were prepared as indicated in Table IV in a 50 percent ethanol solution and dry films cast on a flat quartz plate from 20 microliters of solution. The UV absorbance of each solution was measured.

TABLE IV

| Example | Film-Forming Polymer | Conc. of UV-absorbing Material (1) | UV-absorbing Material | Conc. of Film-Forming Polymer (1) | Absorbance (2) |
|---|---|---|---|---|---|
| 29 | CALGON 502 | 1% | PABA | 1% | 1.2 |
| 30 | CROTEIN Q | 1% | PABA | 1% | 0.26 |
| 31 | METHOCEL | 0.5% | PABA | 0.5% | 1.2 |
| 32 | JR 400 | 0.5% | Ethyl PABA (3) | 0.5% | 0.6 |
| 33 | JR 400 | 1% | Ethyl PABA | 0.5% | 0.9 |

(1) Concentration expressed as weight percents.

(2) Absorbance of a dry film cast from 20 microliters of a solution (50 percent ethanol on 3 $cm^2$ planar quartz surface.

(3) Absorbance of a dry film cast from 20 microliters of a solution (50 percent ethanol on 3 $cm^2$ planar quartz surface) had an absorbance of 0.08.

COMPARATIVE EXAMPLES 34-45

The potentiation of commercial sunscreening compositions was evaluated by adding a film-forming polymer to commercial sunscreening compositions as shown in Table V. In each case the addition of the film-forming polymer was observed to increase the UV absorbance of the sunscreening composition.

TABLE V

| Example | Commercial Product | UV-Absorbing Material | Conc. of UV-Absorbing Material (1) | Film-Forming Polymer | Conc. of Film-Forming Polymer | Absorbance |
|---|---|---|---|---|---|---|
| 34 | PRESUN (GEL)(2) | PABA | 0.5% | - | - | 0.3 |
| 35 | PRESUN (GEL) | PABA | 0.5% | JR-400 | 0.5% | 1.4 |
| 36 | PRESUN (GEL) | PABA | 0.5% | CELLOSIZE QP 300H | 0.5% | 1.4 |
| 37 | PRESUN (LOTION) | PABA | 0.5% | CELLOSIZE QP 300H | 0.5% | 1.3 |
| 38 | PRESUN (LOTION) | PABA | 0.5% | JR-400 | 0.5% | 2.2 |
| 39 | PRESUN (LOTION) | PABA | 0.5% | KLUCEL L | 0.5% | 1.3 |
| 40 | PRESUN (LOTION) | PABA | 0.5% | KLUCEL F | 0.5% | 0.70 |
| 41 | PRESUN (LOTION) | PABA | 0.5% | - | - | 0.16 |
| 42 | ROYAL HAWAIIAN(3) | PABA | 2.5% | - | - | 0.18 |
| 43 | ROYAL HAWAIIAN | PABA | 2.5% | JR 400 | 1.0% | 2.2 |
| 44 | PRESUN LOTION | PABA | 2.5% | - | - | 0.26 |
| 45 | PRESUN LOTION | PABA | 2.5% | JR 400 | 1.0% | 3.3 |

(1) Diluted from 5% PABA in commercial product to 0.5 percent by weight or 2.5 percent by weight, as indicated. (50 percent ethanol).

(2) PRESUN in the trademark of Westwood Phar. Inc. for sunscreening compositions containing 5 percent by weight PABA.

(3) ROYAL HAWAIIAN is the trademark of Royal Hawaiian Paba Corporation for sunscreening compositions containing 5 percent by weight PABA.

EXAMPLE 46

According to this invention a composition was prepared to demonstrate the invention when the topical material is an insecticide.

According to this invention, three compositions were prepared containing the insecticide 1-naphthyl-N-methyl carbamate and one each of the polymers of the group GANTREZ ES-225, HEC(QP330H) and PEO(20M). The compositions had (film-forming polymer) to (1-naphthyl-N-methylcarbamate) weight ratios of 3:1 for HEC(QP330H) and PEO(20M) and 4:1 for GANTREZ ES-225.

Dry films were cast of the aforementioned compositions on an aluminum DSC specimen cup from 50 microliters of solution (100% ethanol as solvent containing about 0.5 or 1. percent by weight of 1-naphthyl-N-methylcarbamate) and the crystallinity of 1-naphthyl-N-methylcarbamate in each composition determined by DSC. DSC showed almost the complete absence of a melting point at about 144°C. This is consistent with a fully dispersed topical material and formation of a microdispersion.

PATENTANWÄLTE
WUESTHOFF - v. PECHMANN - BEHRENS - GOETZ
EUROPEAN PATENT ATTORNEYS

DR.-ING. FRANZ WUESTHOFF
DR. PHIL. FREDA WUESTHOFF (1927-1956)
DIPL.-ING. GERHARD PULS (1952-1971)
DIPL.-CHEM. DR. E. FREIHERR VON PECHMANN
DR.-ING. DIETER BEHRENS
DIPL.-ING.; DIPL.-WIRTSCH.-ING. RUPERT GOETZ

D-8000 MÜNCHEN 90
SCHWEIGERSTRASSE 2
TELEFON: (089) 66 20 51
TELEGRAMM: PROTECTPATENT
TELEX: 524 070



EP-55 423
12,914



WHAT IS CLAIMED IS:

1. A topical composition that forms a dry film when applied to a surface wherein said composition comprises a film-forming polymer in admixture with a topical material wherein the ratio of film-forming polymer to topical material is from about 0.5:1 to about 20:1 such that potentiation of the topical material occurs when said composition is applied to said surface in a dry cast film.

2. The composition of claim 1 wherein the ratio of film-forming polymer to topical material is from about 1:1 to about 10:1 preferably from about 1:1 to about 4:1.

3. A sunscreening composition according to claim 1 or 2 formed by selecting as the topical material an ultraviolet light-absorbing material.

4. The composition of claim 3 wherein the untraviolet light-absorbing material is present in an amount from about 0.1 to about 5.0 percent by weight.

5. The composition of claim 3 or 4 wherein the ultraviolet light-absorbing material is p-amino benzoic acid.

6. The composition of claim 1 to 5 wherein the film-forming polymer is a water-soluble cellulose derivative or an ethylene oxide resin.

7. The sunscreening composition of claim 6 wherein the film-forming polymer is a quaternary nitrogen-substituted cellulose having the structure

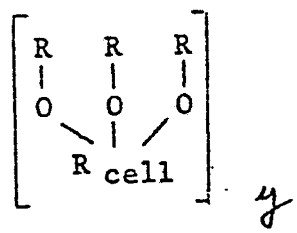

wherein $R_{cell}$ is a residue of an anhydroglucose unit, wherein y is an integer having values 50 to 20,000, and wherein each R individually represents a substituent of the general formula:

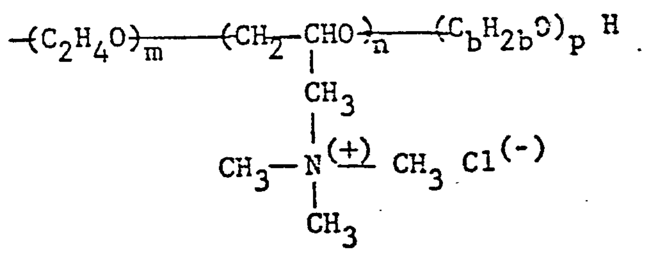

wherein the m is an integer having values from 0 to 10, n is an integer having values from 0 to 3, p is an integer having values from 0 to 10, and b is an integer having a value from 0 to 3 and the average value of n per anhydroglucose unit is from about 0.1 to about 0.5 and the sum (m + p) is from about .8 to about 2.

8. A topical composition according to claim 1 to 6 wherein the film-forming polymer is polyethylene oxide, the topical material is 2-ethyl hexyl para-diethylamino benzoate and the ratic of film-forming polymer to topical material is from about 1:5 to about 4:1.

9. The composition of claim 1 or 2 wherein the topical material is an insecticide preferably 1-naphthyl-N-methylcarbamate.

10. A process for potentiating topically applied materials in dry cast films comprising adding to said topical material a film-forming polymer in such amount that the ratio as a weight percent of film-forming polymer to topical material is from about 0.5:1 to about 20:1 preferably from about 1:1 to about 10:1, especially from about 1:1 to about 4:1 such that the topical material is potentiated in the dry cast film.

11. The process of claim 10 wherein the topical material is an ultraviolet light-absorbing material or an insecticide.

12. A process for producing a shift in the absorbance of ultraviolet light of an ultraviolet light-absorbing material in a dry cast film as compared to the absorbance as measured in a solution of said ultraviolet light absorbing material which comprises adding to a solution of said ultraviolet light-absorbing material a film-forming polymer such that the ratio of film-forming polymer to topical material preferably to ultraviolet absorbing material is from about 0.5:1 to 20:1 preferably about 1:1 to about 4:1 prior to casting said dry film.

13. The process of claim 12 wherein the pH of said solution is adjusted to a pH of from about 4 to about 8 prior to casting said dry film.

14. In a sunscreening composition comprising a UV-absorbing material and a film-forming polymer the improvement of providing said film-forming polymer in an amount such that the weight ratio of film-forming polymer to UV-absorbing material is from about 1:1 to about 4:1 such that the absorbance of ultraviolet light by a dry cast film of said film-forming polymer and said UV-absorbing material is greater than a dry cast film wherein the film-forming polymer is absent.

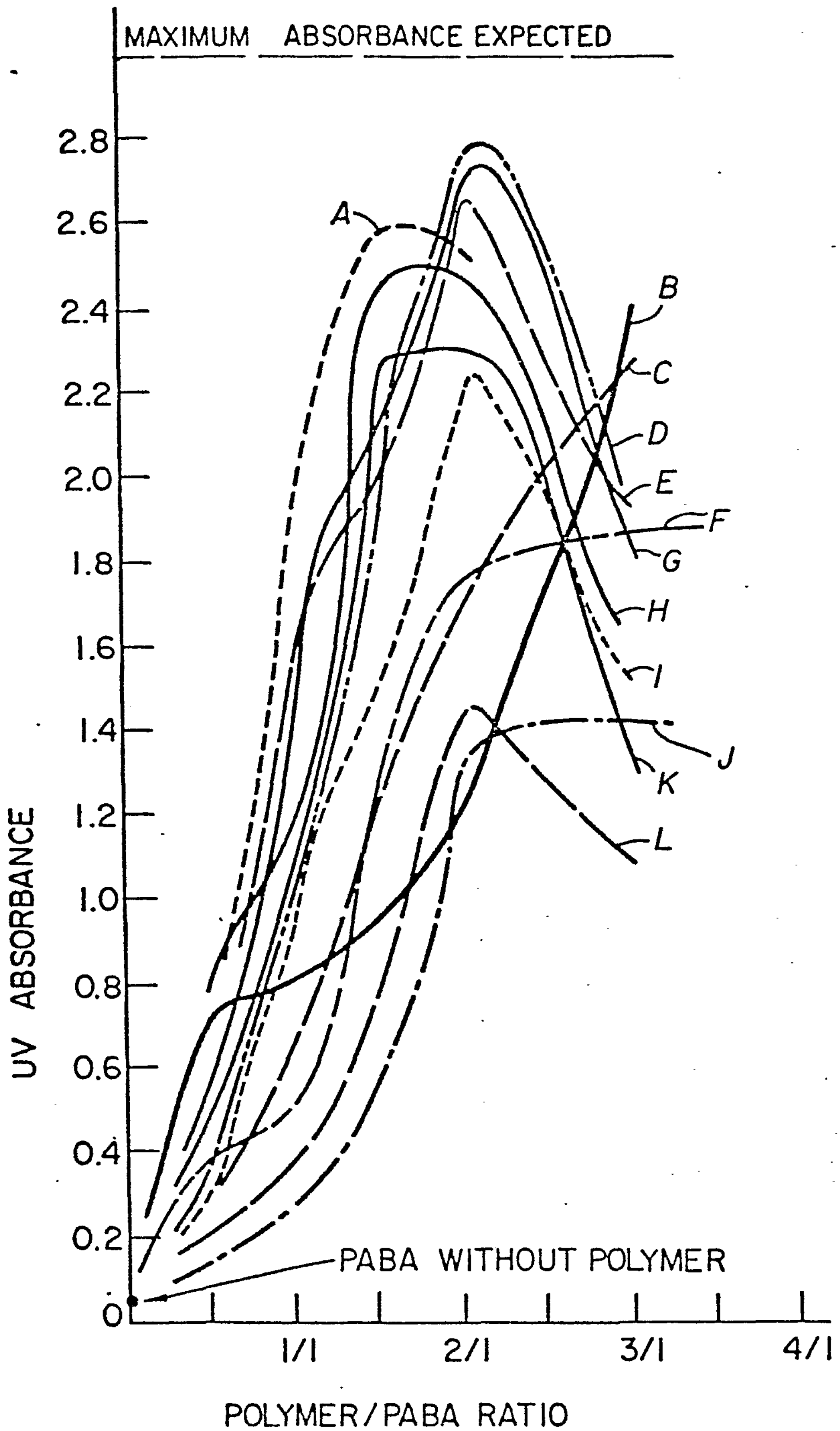
MAXIMUM ABSORBANCE EXPECTED
2.8
2.6
2.4
2.2
2.0
1.8
1.6
1.4
1.2
1.0
0.8
0.6
0.4
0.2
0
UV ABSORBANCE
A
B
C
D
E
F
G
H
I
J
K
L
PABA WITHOUT POLYMER
1/1
2/1
3/1
4/1
POLYMER/PABA RATIO